(19) Europäisches Patentamt
European Patent Office
Office européen des brevets

(11) Veröffentlichungsnummer : **0 123 797**
**B1**

(12) **EUROPÄISCHE PATENTSCHRIFT**

(45) Veröffentlichungstag der Patentschrift : **01.10.86**

(51) Int. Cl.⁴ : **C 08 G 59/58**, C 08 G 59/50, C 09 D 3/58

(21) Anmeldenummer : **84101229.7**

(22) Anmeldetag : **07.02.84**

(54) **Verfahren zur Herstellung von Mattlacken.**

(30) Priorität : **29.03.83 DE 3311404**

(43) Veröffentlichungstag der Anmeldung : **07.11.84 Patentblatt 84/45**

(45) Bekanntmachung des Hinweises auf die Patenterteilung : **01.10.86 Patentblatt 86/40**

(84) Benannte Vertragsstaaten : **AT BE CH DE FR GB IT LI NL SE**

(56) Entgegenhaltungen :
**DE-A- 2 630 011**
**US-A- 4 007 299**

(73) Patentinhaber : **HÜLS AKTIENGESELLSCHAFT**
**Patentabteilung / PB 15 - Postfach 13 20**
**D-4370 Marl 1 (DE)**

(72) Erfinder : **Gude, Fritz, Dr.**
**Wilhelmstrasse 4**
**D-4690 Herne 2 (DE)**
Erfinder : **Haferkorn, Herbert**
**Horster Strasse 528**
**D-4250 Bottrop (DE)**
Erfinder : **Riemer, Heinz, Dr.**
**Fritz-Reuter-Strasse 15**
**D-4250 Bottrop 2 (DE)**
Erfinder : **Dörmann, Günter**
**Händelstrasse 59**
**D-4630 Bochum (DE)**

**EP 0 123 797 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die Herstellung von Salzen aus Polycarbonsäuren mit drei und mehr Carboxylgruppen und cyclischen Amidinen in organischen Lösungsmitteln zum Zwecke der Härtung von Epoxidharzen zu matten Überzügen wird in der DE-B-23 24 696 beschrieben. So führt beispielsweise die Umsetzung von einem Mol Pyromellithsäure mit 2 Molen 2-Phenyl-Δ2-imidazolin in Lösungsmitteln, wie Dimethylformamid, Dimethylacetamid, N-Methylpyrrolidon, aliphatischen Ketonen oder auch Alkoholen zu dem Disalz der Pyromellithsäure, das wegen seiner Schwerlöslichkeit ausfällt.

In gleicher Weise erhält man in den oben genannten Lösungsmitteln nach ebenfalls bekannter Verfahrensweise durch Zusammenfügen von Pyromellithsäure und 2-Phenyl-Δ2-imidazolin im Molverhältnis 1 : 1 das Monosalz.

Beide Salze sind gemäß DE-B-23 24 696 Härter für Epoxidharze zur Herstellung von Überzügen mit besonders wertvollen Eigenschaften. So können bei Verwendung des Disalzes der Pyromellithsäure mit 2-Phenyl-Δ2-imidazolin als Härter für Epoxidharze matte Überzüge mit besonders guten mechanischen Werten erhalten werden. Das Monosalz aus diesen Komponenten führt zu noch geringeren Glanzgraden ; allerdings fallen damit die mechanischen Eigenschaften der Filme ab, so daß sich schon relativ geringe Mengen Monosalz im Disalz in dieser Hinsicht sehr negativ bemerkbar machen. Nachteilig ist die Verwendung von organischen Lösungsmitteln bei der Herstellung der Salze. Man benötigt dazu sowohl zu ihrer Herstellung als auch zur Aufarbeitung geschlossene Apparaturen, hat mit giftigen und brennbaren Flüssigkeiten zu arbeiten und muß nach der Reaktion die Lösungsmittel durch Destillation reinigen, um sie wieder in den Verfahrenskreislauf zurückführen zu können. Es hat deshalb nicht an Versuchen gefehlt, die organischen Lösungsmittel durch Wasser zu ersetzen. Nachteilig ist hierbei u. a. die wesentlich geringere Löslichkeit des 2-Phenyl-Δ2-imidazolins in Wasser als in den oben beschriebenen Lösungsmitteln. Man geht deshalb normalerweise so vor, daß man Pyromellithsäure in Wasser vorlegt und durch Aufheizen auf 70 bis 80 °C eine klare Lösung erstellt, wonach man die dem Mono- bzw. Disalz entsprechende stöchiometrische Menge festes 2-Phenyl-Δ2-imidazolin unter Rühren einträgt. Nach etwa 2 Stunden Rühren bei rund 90 °C und Abkühlen auf Raumtemperatur wird das ausgefallene Salz durch Filtration gewonnen.

Bei der anwendungstechnischen Untersuchung dieser, auf diese Weise gewonnenen Salze der Pyromellithsäure, stellte sich heraus, daß das am schwersten lösliche Monosalz nur dann dem aus organischen Lösungsmitteln gefällten Monosalz identische Werte ergibt, wenn man es, wie in der DE-A-30 26 455 beschrieben, in Teilchengrößen $< 60$ µm einsetzt.

Im Gegensatz zu dem Monosalz war es nicht möglich, das Disalz reproduzierbar und optimal aus Wasser herzustellen. Die mechanischen Eigenschaften der mit Epoxidharz nach DE-B-23 24 696 hergestellten Überzüge konnten dann nicht mehr erreicht werden. Eine Differentialthermoanalyse des in Wasser hergestellten Disalzes ergab, daß es durch Monosalz verunreinigt war.

Nach der oben geschilderten Herstellungsweise ist also die Anwesenheit von Monosalz im Disalz bei Fällungen aus Wasser nicht zu vermeiden. Es konnte experimentell weiter festgestellt werden, daß sich das Monosalz infolge seiner Schwerlöslichkeit in Wasser nur unvollkommen in das Disalz überführen läßt, wenn man zu der Aufschlämmung von einem Mol Monosalz in Wasser ein Mol 2-Phenyl-Δ2-imidazolin zugibt. Die Reaktion ist selbst bei 12 bis 15-stündigem Kochen dieser beiden Komponenten in Wasser noch nicht vollständig, und man muß unter diesen Bedingungen mit teilweiser hydrolytischer Aufspaltung des Imidazolinringes rechnen.

Es stellte sich nun überraschend heraus, daß die gleichen guten mechanischen Eigenschaften und gleicher Mattglanzgrad gehärteter Epoxidharzschichten erreicht werden können, wenn man anstelle des aus organischen Lösungsmitteln gefällten Disalzes aus 2-Phenyl-Δ2-imidazolin und Pyromellithsäure ein Monosalz aus 2-Phenyl-methyl-Δ2-imidazolin und Trimellithsäure als Härter verwendet, dessen Herstellung in Wasser erfolgt. Im Gegensatz zu dem Disalz aus 2-Phenyl-Δ2-imidazolin und Pyromellithsäure ist das Monosalz aus 2-Phenyl-methyl-Δ2-imidazolin und Trimellithsäure nicht hygroskopisch.

Das 2-Phenyl-methyl-Δ2-imidazolin wird durch Kondensation von 1,2-Propandiamin mit Benzonitril, Benzoesäure oder Benzoesäureestern in bekannter Weise hergestellt. Bei der Untersuchung der Substanz durch Kernresonanzspektroskopie stellte sich heraus, daß hier kein Isomerengemisch, sondern eine einheitliche Substanz vorliegt. Die Stellung der Methyl- zur C=N-Gruppe kann allerdings nicht festgelegt werden. Es liegt eine Tautomerie vor :

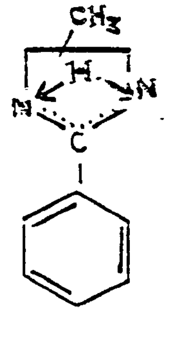

Gegenstand des vorliegenden Schutzrechtes ist daher ein Verfahren zur Herstellung von Mattlacken auf der Basis von Pulverbeschichtungen aus 1,2-Epoxidverbindungen mit mindestens einer 1,2-Epoxidgruppe im Molekül und einem unteren Aufschmelzpunkt von > 40 °C, Salzen aus aromatischen Carbonsäuren mit 3 und mehr Carboxylgruppen und Imidazolinen als Härter sowie üblichen Zusätzen und Aufbringen des Pulverlackes auf die zu lackierenden Gegenstände, wobei diese auf Temperaturen von 160-240 °C erhitzt werden, das dadurch gekennzeichnet ist, daß man als Härter das Salz aus 2-Phenyl-methyl-Δ2-imidazolin und Trimellithsäure im Molverhältnis 1 : 1 und als Lösungsmittel zur Herstellung des Salzes Wasser verwendet.

Wie sich weiter überraschend herausstellte, ist es ohne Qualitätseinbuße der daraus hergestellten Mattlacke möglich, das 2-Phenyl-methyl-Δ2-imidazolin sogar bis zu 60 Mol% durch 2-Phenyl-Δ2-imidazolin zu ersetzen.

## Beispiele

### A) Herstellung der Monosalze

1) Monosalz aus Trimellithsäure und 2-Phenyl-methyl-Δ2-imidazolin (im folgenden kurz « Phenyl-methyl-imidazolin » genannt).

0,5 Mol Trimellithsäureanhydrid heizt man in 750 ml Wasser auf etwa 90 °C, wobei Hydrolyse und Lösung eintritt. Nun wird 0,5 Mol Phenyl-methyl-imidazolin bei gleicher Temperatur zudosiert. Nach etwa 5 Minuten beginnt die Ausfällung des Salzes, die durch Abkühlen auf Zimmertemperatur vervollständigt wird. Nach Abtrennen und Trocknen des feinkristallinen farblosen Niederschlages erhält man die unmittelbar einsetzbare Substanz mit einer Ausbeute von 98 % der Theorie.

2) Monosalz-Gemische aus Trimellithsäure und Phenyl-methyl-imidazolin und 2-Phenyl-Δ2-imidazolin

Die Salzgemische können sinngemäß wie unter A1) beschrieben hergestellt werden. Es ist auch möglich, die entsprechenden Monosalze getrennt zu fällen, zu zerkleinern und intensiv miteinander zu mischen.

### B) Herstellung der Mattlacke

1) das Monosalz aus Trimellithsäure und Phenyl-methyl-imidazolin wurde mit Titandioxid und dem angegebenen Epoxidharz sowie einem Zusatz an Verlaufmittel in folgendem Verhältnis zu Pulverlack verarbeitet :

| | |
|---|---|
| Festes Epoxidharz, auf der Basis eines Adduktes aus 2,2-Bis(4-hydroxy-phenyl)-propan(Dian) und Epichlorhydrin, welches einer HCl-Abspaltung unterworfen und anschließend mit weiterem Dian umgesetzt wurde, und welches nach Angabe des Herstellers ein Epoxid-Äquivalentgewicht von 900-1 000 besitzt, was einem Epoxidwert von 0,10-0,11 entspricht und einem Schmelzbereich von 90-100°C : | 54,0 Gew.% |
| Monosalz aus Trimellithsäure und Phenyl-methyl-imidazolin (Härter) : | 5,0 Gew.% |
| $TiO_2$ (in Pulverform) | 40,0 Gew.% |
| Verlaufmittel (« Modaflow Powder® ») : | 1,0 Gew.% |

Diese Formulierung wurde auf die Testbleche aufgebracht und 10 Minuten bei 200 °C gehärtet. Die anschließende Prüfung ergab folgende Werte :

| | |
|---|---|
| Schichtdicke | 50-60 μm |
| Glanzgrad (n. Gardner 60°) (ASTM D-523-53 T) | 24 % |
| Erichsentiefung (DIN 53156) | 6,8 mm |
| Gitterschnitt (DIN 53151) | Gt O |
| Dornbiegeversuch (DIN 53152) | < 2 mm |
| Buchholzhärte (DIN 50153) | 111 |
| Kugelschlag (n. Gardner) direct impact (ASTM D-2794) | > 2 kg · m |

2) Eine Mischung der Monosalze von Trimellithsäure mit 2-Phenyl-imidazolin und Phenyl-methyl-imidazolin, in der die beiden Monosalze im Molverhältnis von 10 : 90 vorliegen, wurde als Härter in der Rezeptur des Beispiels 1 verwendet.

Nach 10-minütiger Härtung bei 200 °C wurden folgende lacktechnische Werte erhalten :

| | |
|---|---|
| Schichtdicke | 55-60 μm |
| Glanzgrad (n. Gardner 60°) (ASTM D-523-53 T) | 20 % |
| Erichsentiefung (DIN 53156) | 7,2 mm |
| Gitterschnitt (DIN 53151) | Gt O |
| Dornbiegeversuch (DIN 53152) | < 2 mm |

| | |
|---|---|
| Buchholzhärte (DIN 53153) | 111 |
| Kugelschlag (n. Gardner) direct impact (ASTM D-2794) | > 2 kg · m |

3) Die Mischung der Monosalze entsprechend Beispiel 2, jedoch im Verhältnis 20 : 80, wurde in die Rezeptur des Beispiels 1 eingesetzt.

Nach 10-minütiger Härtung bei 200 °C wurden folgende lacktechnische Werte erhalten :

| | |
|---|---|
| Schichtdicke | 50-60 μm |
| Glanzgrad (n. Gardner 60°) (ASTM D-523-53 T) | 22 % |
| Erichsentiefung (DIN 53156) | 6,8 mm |
| Gitterschnitt (DIN 53151) | Gt O |
| Dornbiegeversuch (DIN 53152) | < 2 mm |
| Buchholzhärte (DIN 53153) | 100 |
| Kugelschlag (n. Gardner) direct impact (ASTM D-2794) | > 2 kg · m |

4) Die Mischung der Monosalze entsprechend den Beispielen 2 und 3, jedoch im Verhältnis 30 : 70, wurde als Härter in der Rezeptur des Beispiels 1 verwendet.

Nach 10-minütiger Härtung bei 200 °C wurden folgende Testergebnisse ermittelt :

| | |
|---|---|
| Schichtdicke | 60-65 μm |
| Glanzgrad (n. Gardner 60°) (ASTM D-523-53 T) | 24 % |
| Erichsentiefung (DIN 53156) | 7,3 mm |
| Gitterschnitt (DIN 53151) | Gt O |
| Dornbiegeversuch (DIN 53152) | < 2 mm |
| Buchholzhärte (DIN 53153) | 111 |
| Kugelschlag (n. Gardner) direct impact (ASTM D-2794) | > 2 kg · m |

5) Die Mischung der Monosalze entsprechend den Beispielen 2, 3 und 4, jedoch im Verhältnis 40 : 60, wurde als Härter in die Rezeptur des Beispiels 1 eingesetzt.

Nach 15-minütiger Härtung bei 180 °C wurden folgende lacktechnische Werte ermittelt :

| | |
|---|---|
| Schichtdicke | 55-60 μm |
| Glanzgrad (n. Gardner 60°) (ASTM D-523-53 T) | 23 % |
| Erichsentiefung (DIN 53156) | 7,8 mm |
| Gitterschnitt (DIN 53151) | Gt O |
| Dornbiegeversuch (DIN 53152) | < 2 mm |
| Buchholzhärte (DIN 53153) | 100 |
| Kugelschlag (n. Gardner) direct impact (ASTM D-2794) | > 2 kg · m |

**Patentansprüche**

1. Verfahren zur Herstellung von Mattlacken auf der Basis von Pulverbeschichtungen aus 1,2-Epoxidverbindungen mit mindestens einer 1,2-Epoxidgruppe im Molekül und einem unteren Aufschmelzpunkt von > 40 °C, Salzen aus aromatischen Carbonsäuren mit 3 und mehr Carboxylgruppen und Imidazolinen als Härter, sowie üblichen Zusätzen und Aufbringen des Pulverlackes auf die zu lackierenden Gegenstände, wobei diese auf Temperaturen von 160-240 °C erhitzt werden, dadurch gekennzeichnet, daß man als Härter das Salz aus 2-Phenyl-methyl-Δ2-imidazolin und Trimellithsäure im Molverhältnis 1 : 1 und Wasser als Lösungsmittel zur Herstellung des Salzes verwendet.

2. Verfahren nach Anspruch 1, dadurch gekennzeichnet, daß das verwendete 2-Phenyl-methyl-Δ2-imidazolin aus Benzonitril und 1,2-Propandiamin hergestellt wird.

3. Verfahren nach den Ansprüchen 1 und 2, dadurch gekennzeichnet, daß man das Salz aus 2-Phenyl-methyl-Δ2-imidazolin und Trimellithsäure bis zu 60 Mol% durch ein Salz aus 2-Phenyl-Δ2-imidazolin und Trimellithsäure ersetzt.

**Claims**

1. Process for the preparation of flat paints based on powder coatings consisting of 1,2-epoxide compounds having at least one 1,2-epoxide group in the molecule and a lower fusion point of > 40 °C, salts obtained from aromatic carboxylic acids having 3 or more carboxyl groups and imidazolines as a hardener and conventional additives, and application of the powder coating to the objects which are to be painted, the latter being heated to temperatures of 160-240 °C, characterized in that the salt of 2-phenyl-methyl-Δ2-imidazoline and trimellitic acid in a molar ratio of 1 : 1 is used as a hardener and water is used as the solvent for the preparation of the salt.

2. Process according to Claim 1, characterized in that the 2-phenyl-methyl-Δ2-imidazoline used is prepared from benzonitrile and 1,2-propanediamine.

3. Process according to Claims 1 and 2, characterized in that the salt of 2-phenyl-methyl-Δ2-imidazoline and trimellitic acid is replaced up to an extent of 60 mol% by a salt of 2-phenyl-Δ2-imidazoline and trimellitic acid.

**Revendications**

1. Procédé pour la réalisation de vernis mats à base de couches pulvérulentes constituées de combinaisons époxy 1,2 avec au moins un groupe époxy 1,2 dans la molécule et un point inférieur de fusion supérieur à 40 °C, de sels d'acides de carbone aromatiques avec 3 groupes carboxyle ou davantage, et avec comme durcisseurs des imidazolines, ainsi que des ajouts classiques, et pour le dépôt du vernis pulvérulent sur les objets à vernir, ceux-ci étant chauffés à des températures de 160 à 240 °C, procédé caractérisé, en ce qu'on utilise comme durcisseur le sel constitué de 2-phényl-méthyl-Δ2-imidazoline et d'acide trimellitique en rapport moléculaire 1 : 1, ainsi que de l'eau comme solvant pour la production du sel.

2. Procédé selon la revendication 1, caractérisé en ce que la 2-phényl-méthyl-Δ2-imidazoline utilisée est obtenue à partir de benzonitrile et de diamine 1,2 de propane.

3. Procédé selon les revendications 1 et 2, caractérisé en ce qu'on remplace le sel constitué de 2-phényl-méthyl-Δ2-imidazoline et d'acide trimellitique, jusqu'à 60 % en molécules-grammes, par un sel constitué de 2-phényl-Δ2-imidazoline et d'acide trimellitique.